# EUROPÄISCHE PATENTSCHRIFT

(11) **EP 3 973 053 B1**
(45) Veröffentlichungstag und Bekanntmachung des Hinweises auf die Patenterteilung: **18.01.2023**
(21) Anmeldenummer: 19726935.0
(22) Anmeldetag: 20.05.2019
(51) Int. Cl.: C12N 9/00, C12P 21/02

(54) **MIKROORGANISMEN-STAMM UND VERFAHREN ZUR FERMENTATIVEN HERSTELLUNG VON GAMMA-GLUTAMYLTYROSIN UND GAMMA-GLUTAMYLPHENYLALANIN**
STRAIN OF MICROORGANISMS AND PROCESS FOR THE FERMENTATIVE PRODUCTION OF GAMMA-GLUTAMYLTYROSINE AND GAMMA-GLUTAMYLPHENYLALANINE
SOUCHE DE MICRO-ORGANISMES ET PROCÉDÉ DE PRODUCTION DE GAMMA-GLUTAMYLTYROSINE ET DE GAMMA-GLUTAMYLPHÉNYLALANINE PAR FERMENTATION

(43) Veröffentlichungstag der Anmeldung: 30.03.2022
(73) Patentinhaber: Wacker Chemie AG, 81737 München (DE)
(72) Erfinder: SCHLÖSSER, Thomas, 84489 Burghausen (DE); THÖN, Marcel, 06114 Halle (DE)
(74) Vertreter: Belz, Ferdinand
(86) Internationale Anmeldenummer: PCT/EP2019/062957
(87) Internationale Veröffentlichungsnummer: WO 2020/233784

(56) Entgegenhaltungen:
- JP-B2- 4 089 164
- US-A1- 2014 342 399
- B. S. KELLY ET AL: "Escherichia coli ?-Glutamylcysteine Synthetase: TWO ACTIVE SITE METAL IONS AFFECT SUBSTRATE AND INHIBITOR BINDING", JOURNAL OF BIOLOGICAL CHEMISTRY, Bd. 277, Nr. 1, 4. Januar 2002 (2002-01-04), Seiten 50-58, XP055217374, ISSN: 0021-9258, DOI: 10.1074/jbc.M107961200
- REIKO NAKAYAMA ET AL: "Synthesis of Î3-Glutamylpeptides by Î3-Glutamylcysteine Synthetase from Proteus mirabilis", AGRICULTURAL AND BIOLOGICAL CHEMISTRY,, Bd. 45, Nr. 12, 1. Dezember 1981 (1981-12-01), Seiten 2839-2845, XP001319870,
- HIDEHIKO KUMAGAI ET AL: "Syntheses of y-Glutamyl Peptides by y-Glutamyltranspeptidase from E. coli", ANN. N. Y. ACAD. SCI., Bd. 613, 1. Januar 1990 (1990-01-01), Seiten 647-651, XP055147522, DOI: 10.1111/j.1749-6632.1990.tb18238.x

## Beschreibung

Die Erfindung betrifft ein Verfahren zur fermentativen Herstellung der Substanzen Gamma-Glutamyltyrosin (y-Glu-Tyr) und Gamma-Glutamylphenylalanin (y-Glu-Phe).

Der Begriff Kokumi stammt aus der japanischen Sprache und bezeichnet keinen eigenen Geschmack wie süß, sauer, bitter, salzig oder umami (schmackhaft/würzig), sondern ein Geschmackserlebnis mit stark ausgeprägtem Mundgefühl und anhaltendem Geschmacksreichtum. Zuerst wurde der Kokumi-Effekt von Ueda et al. im Jahr 1990 (Agri. Biol. Chem. 54, S. 163-169) beschrieben, als wässrige Knoblauch-Extrakte in verschiedenen Suppen sensorisch bewertet wurden.

Molekular betrachtet konnte von Ohsu et al. (2010, J. Biol. Chem. 285, S. 1016-1022)) gezeigt werden, dass der Kokumi-Effekt auf der Modulation eines *calcium-sensing* Rezeptors (CaSR) durch Gamma-Glutamyl-Peptide wie Glutathion (y-Glu-Cys-Gly) oder γ-Glu-Val-Gly beruht.

Interessanterweise sind jedoch nicht nur Gamma-Glutamyl-Tripeptide für das Kokumi-Geschmackserlebnis verantwortlich, sondern auch entsprechende Dipeptide. So wurde von Shibata et al. im Jahr 2017 (Biosci. Biotechnol. Biochem. 81, Seiten 2168-2177) gefunden, dass Hitze-behandelte Sojabohnen zusammen mit Glutamat, Salz und Inosin-Monophosphat ein besonders intensives Kokumi-Geschmackserlebnis hervorrufen. Interessanterweise sind die Dipeptide γ-Glu-Tyr und γ-Glu-Phe für den intensiven Kokumi-Effekt verantwortlich. So ist es nicht verwunderlich, dass in würzigem Comte Käse von Roudot-Algaron et al. bereits 1994 γ-Glu-Tyr und γ-Glu-Phe nachgewiesen wurden (J. Dairy Sci. 77, Seiten 1161-1166).

Aufgabe der vorliegenden Erfindung ist die Bereitstellung eines Fermentationsverfahrens zur Herstellung von Gamma-Glutamyltyrosin (y-Glu-Tyr) und Gamma-Glutamylphenylalanin (γ-Glu-Phe).

Diese Aufgabe wird gelöst durch ein Verfahren zur fermentativen Produktion von Gamma-Glutamyltyrosin und Gamma-Glutamylphenylalanin, dadurch gekennzeichnet, dass ein Mikroorganismenstamm in einem Fermentationsmedium kultiviert, das Fermentationsmedium nach der Fermentation von den Zellen abgetrennt und Gamma-Glutamyltyrosin und Gamma-Glutamylphenylalanin aus dem Fermentationsmedium isoliert werden, wobei der Mikroorganismenstamm mindestens ein weiteres Gen codierend eine Glutamat-Cystein-Ligase enthält und defizient in der Glutathion-Synthethase ist.

Als Ausgangsstämme sind prinzipiell alle Mikroorganismenstämme geeignet, die rekombinanten Verfahren zugänglich sind und durch Fermentation kultivierbar sind. Solche Mikroorganismenstämme können Pilze, Hefen oder Bakterien sein. Bevorzugt handelt es sich beim Mikroorganismenstamm um einen Bakterienstamm, besonders bevorzugt um einen Bakterienstamm der phylogenetischen Gruppe der Eubacteria. Besonders bevorzugt sind Mikroorganismenstämme der Familie Enterobacteriaceae und insbesondere bevorzugt handelt sich beim Bakterienstamm um einen Stamm der Art *Escherichia coli (E. coli).*

Bevorzugt sind E. coli-Stämme, die zur Produktion von Gamma-Glutamylcystein geeignet sind. Die Konstruktion solcher Stämme ist in der Patentschrift US 2014/0342399 beschrieben. Besonders bevorzugt ist der in US 2014/0342399 A offenbarte E. coli-Stamm W3110ΔgshB/ptufBₚ-gshA^{ATG}-cysE14-serA2040-orf306.

Als offener Leserahmen (*open reading frame,* ORF) wird derjenige Bereich der DNS bzw. RNS bezeichnet, der mit dem Startcodon beginnt und bis zu einem Stoppcodon führt und für die Aminosäuresequenz eines Proteins codiert. Der ORF wird synonym auch als codierende Sequenz bezeichnet.

ORFs werden von nicht-codierenden Bereichen umgeben. Als Gen wird der DNS-Abschnitt bezeichnet, der alle Grundinformationen zur Herstellung einer biologisch aktiven RNS enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle. Des Weiteren sind als Expressionssignale ein Terminator und ein oder mehrere Operatoren möglich.

Für einen funktionellen Promotor gilt, dass der unter der Regulation dieses Promotors stehende ORF in eine RNS transkribiert wird.

Erfindungsgemäß enthält der Mikroorganismenstamm mindestens einen weiteren ORF codierend eine Glutamat-Cystein-Ligase. Der Begriff "ein weiterer ORF" bezieht sich darauf, dass ein ORF codierend eine Glutamat-Cystein-Ligase bereits im Wildtyp-Genom vorhanden ist und dieser chromosomale ORF nicht gemeint ist.

Im Rahmen dieser Erfindung bedeutet der Begriff "ein weiterer ORF" dagegen, dass:
a) entweder ein bezüglich des Glutamat-Cystein-Ligase-ORFs Wildtyp-Mikroorganismenstamm vorliegt, d.h. dieser enthält den im Wildtyp-Genom codierten ORF codierend die Glutamat-Cystein-Ligase und zusätzlich mindestens einen weiteren ORF codierend eine Glutamat-Cystein-Ligase,
b) oder der erfindungsgemäße Mikrooorganismenstamm besitzt im Wildtyp-Genom keinen ORF codierend die Glutamat-Cystein-Ligase, aber mindestens einen weiteren ORF codierend eine Glutamat-Cystein-Ligase, d.h. der erfindungsgemäße Mikroorganismenstamm exprimiert das Enzym nur von dem oder den weiteren ORF(s).

Die Glutamat-Cystein-Ligase oder Gamma-Glutamylcystein-Synthethase ist ein Enzym, das im Stoffwechsel aus den Edukten L-Cystein und L-Glutamat unter Verbrauch eines Moleküls ATP Gamma-Glutamylcystein herstellt. Dieses Enzym wird von einem Gen codiert, das beispielsweise in *E. coli* mit gshA und in *Saccharomyces cerevisiae* (*S. cerevisiae*) mit GSH1 bezeichnet wird. Bevorzugt handelt es sich bei der Glutamat-Cystein-Ligase um das von der codierenden Region des gshA-Gens (gshA-ORF) codierte Genprodukt GshA oder GshA-Protein. Bevorzugt handelt es sich beim weiteren ORF codierend eine Glutamat-Cystein-Ligase um SEQ ID NO. 1 oder Homologe dieser Sequenz, wobei bevorzugt das Startcodon ATG ist, besonders bevorzugt handelt es sich um SEQ ID NO. 1.

Im Rahmen der vorliegenden Erfindung sind Homologe auf DNS-Ebene solche ORFs, die eine Sequenzidentität größer 30 %, besonders bevorzugt größer 70 %, zur im jeweiligen Mikroorganismus in seiner Wildtyp-Form vorhandenen ORF-Sequenz codierend eine Glutamat-Cystein-Ligase, in *E. coli* insbesondere bevorzugt zu SEQ ID NO. 1 besitzen und wobei das von dieser DNS-Sequenz exprimierte Protein Glutamat-Cystein-Ligase-Funktion aufweist (Definition und Nachweis von funktioneller Glutamat-Cystein-Ligase, d.h. dem Vorliegen eines Proteins mit Glutamat-Cystein-Ligase-Funktion s. unten).

Die Bestimmung von Sequenzidentitäten von DNA-Sequenzen erfolgt durch das Programm "nucleotide blast", zu finden auf der Seite http://blast.ncbi.nlm.nih.gov/, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind:
Max target sequences = 100;
Short queries = "Automatically adjust parameters for short input sequences";
Expect Threshold = 10;
Word size = 28;
Automatically adjust parameters for short input sequences = 0.

Die entsprechenden voreingestellten Scoring Parameter sind:
Match/Mismatch Scores = 1,-2;
Gap Costs = Linear.

Bevorzugt handelt es sich beim GshA-Protein um ein Protein mit SEQ ID NO. 2 und Homologe dieser Sequenz, besonders bevorzugt um ein Protein mit SEQ ID NO. 2.

Homologe auf Proteinebene sind Proteine mit einer Sequenzidentität größer 30 %, besonders bevorzugt größer 70 %, zur im jeweiligen Mikroorganismus in seiner Wildtyp-Form vorhandenen Proteinsequenz der Glutamat-Cystein-Ligase, in *E. coli* insbesondere bevorzugt zu SEQ ID NO. 2 und wobei das Protein Glutamat-Cystein-Ligase-Funktion aufweist (Definition und Nachweis von funktioneller Glutamat-Cystein-Ligase, d.h. dem Vorliegen eines Proteins mit Glutamat-Cystein-Ligase-Funktion s. unten).

Für die Bestimmung von Sequenzidentitäten von Proteinsequenzen wird das Programm "protein blast", auf der Seite http://blast.ncbi.nlm.nih.gov/, genutzt.

Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind:
Max target sequences = 100;
Short queries = "Automatically adjust parameters for short input sequences";
Expect Threshold = 10;
Word size = 3;
Automatically adjust parameters for short input sequences = 0.

Die voreingestellten Scoring Parameter sind:
Matrix = BLOSUM62;
Gap Costs = Existence: 11 Extension: 1;
Compositional adjustments = Conditional compositional score matrix adjustment.

Die Funktion des Glutamat-Cystein-Ligase-Proteins in einem erfindungsgemäßen Mikroorganismenstamm kann wie folgt charakterisiert werden:
a) Entweder wird das Vorhandensein von funktioneller Glutamat-Cystein-Ligase im Mikroorganismenstamm indirekt über das Vorhandensein der Produkte γ-Glu-Tyr und γ-Glu-Phe nachgewiesen. Zum Nachweis von γ-Glu-Tyr und γ-Glu-Phe kann die in Beispiel 4 beschriebene HPLC-MS-Methode verwendet werden.
b) Oder das Vorhandensein von funktioneller Glutamat-Cystein-Ligase im Mikroorganismenstamm wird direkt über die Enzymfunktion in einem *in vitro* Test nachgewiesen. Ein Enzymtest zum Nachweis von Glutamat-Cystein-Ligase-Aktivität ist in der Patentschrift US 2014/342399 im Beispiel 9 beschrieben.

Ziel der Erfindung ist es, eine erhöhtezelluläre Aktivität der Glutamat-Cystein-Ligase zu erreichen. Diese wird erfindungsgemäß durch eine verstärkte Expression der Glutamat-Cystein-Ligase und die wiederum durch die Erhöhung der Kopienzahl des ORFs codierend eine Glutamat-Cystein-Ligase erreicht.

Darüber hinaus wird die Expression des ORFs codierend eine Glutamat-Cystein-Ligase bevorzugt durch den Einsatz geeigneter Promotoren erhöht.

Ein Promotor, der zu einer verstärkten Proteinexpression führt, wird als starker Promotor bezeichnet. Ein starker Promotor kann zur Expression des im Wildtyp chromosomal vorhandenen ORFs codierend eine Glutamat-Cystein-Ligase in das entsprechende Wildtyp-Gen kloniert werden und/oder die Expression des zusätzlichen bzw. weiteren(r) ORFs codierend eine Glutamat-Cystein-Ligase regulieren.

Beispiele für Promotoren, die im Mikroorganismus zu einer starken Expression führen (starke Promotoren) sind dem Fachmann bekannt.

In einer bevorzugten Ausführungsform handelt es sich beim erfindungsgemäßen Mikroorganismenstamm um einen bezüglich des Glutamat-Cystein-Ligase-ORFs Wildtyp-Mikroorganismenstamm, d.h. dieser enthält den im Wildtyp-Genom codierten ORF codierend die Glutamat-Cystein-Ligase, und dieser enthält zusätzlich mindestens einen weiteren Plasmid-codierten ORF codierend eine Glutamat-Cystein-Ligase unter Kontrolle eines starken Promotors.

Unter verstärkter Expression des Proteins ist zu verstehen, dass das Protein in erhöhter Ausbeute produziert wird, was bedeutet, dass mit dem erfindungsgemäßen Mikroorganismenstamm verglichen mit einem entsprechenden Wildtyp-Stamm bevorzugt mindestens 110 %, besonders bevorzugt mindestens 150 % und insbesondere bevorzugt mindestens 200 % der Menge an Glutamat-Cystein-Ligase produziert wird. Das heißt, die Ausbeute an Glutamat-Cystein-Ligase-Protein ist bevorzugt mindestens 1,1-mal, besonders bevorzugt mindestens 1,5-mal und insbesondere bevorzugt mindestens 2-mal so hoch wie die Ausbeute, die mit entsprechenden Stämmen, die keinen weiteren offenen Leserahmen codierend eine Glutamat-Cystein-Ligase enthalten, erreicht werden kann. Der entsprechende Wildtyp-Mikroorganismenstamm ist dadurch charakterisiert, dass er genetisch zum verwendeten Stamm identisch ist, aber keinen weiteren offenen Leserahmen codierend eine Glutamat-Cystein-Ligase enthält.

Der erfindungsgemäße Mikroorganismenstamm kann eine oder mehrere weitere funktionelle Kopien des ORFs codierend eine Glutamat-Cystein-Ligase umfassen. Bevorzugt umfasst er eine bis 700 weitere funktionelle Kopien des ORFs codierend eine Glutamat-Cystein-Ligase, besonders bevorzugt eine bis 20 weitere funktionelle Kopien des ORFs codierend eine Glutamat-Cystein-Ligase. Jeder weitere bzw. zusätzliche ORF kann in das Chromosom integriert sein, er kann jedoch auch auf einem oder mehreren Plasmiden vorliegen.

Erfindungsgemäß ist der Mikroorganismenstamm defizient in der Glutathion-Synthethase, d.h. vom entsprechenden Gen wird keine funktionelle Glutathion-Synthethase produziert. Bei der Glutathion-Synthethase handelt es sich bevorzugt um das in *E. coli* vom gshB-Gen und in *S. cerevisiae* vom GSH2-Gen codierte Genprodukt. Bei der Glutathion-Synthethase handelt es sich besonders bevorzugt um das in *E. coli* vom gshB-Gen codierte Genprodukt.

In einer bevorzugten Ausführungsform enthält der Mikroorganismenstamm mindestens ein Plasmid, das mindestens einen, besonders bevorzugt genau einen ORF codierend eine Glutamat-Cystein-Ligase unter Kontrolle eines funktionellen Promotors enthält. In dieser Ausführungsform erfolgt die Expression des ORFs codierend eine Glutamat-Cystein-Ligase auf einem Plasmid. Ein derartiges Produktionsplasmid ermöglicht die Herstellung von γ-Glu-Tyr und γ-Glu-Phe in einem Mikroorganismenstamm.

Es ist bevorzugt, dass der Mikroorganismenstamm das chromosomale Gen codierend eine Glutamat-Cystein-Ligase besitzt. Es ist besonders bevorzugt, dass der Mikroorganismenstamm das chromosomale Gen und mindestens einen weiteren ORF codierend eine Glutamat-Cystein-Ligase besitzt, der auf einem Plasmid lokalisiert ist. Das bedeutet, dass die Zellen zusätzlich zur vom chromosomalen Gen produzierten Glutamat-Cystein-Ligase Plasmidcodierte Glutamat-Cystein-Ligase produzieren.

In einer alternativ bevorzugten Ausführungsform wird ein Mikroorganismenstamm verwendet, bei dem das im Wildtyp vorhandene chromosomale Gen codierend eine Glutamat-Cystein-Ligase deletiert ist und ein bis mehrere zusätzliche(s) ORFs codierend eine Glutamat-Cystein-Ligase (e) chromosomal integriert oder über ein bis mehrere Plasmid(e) eingebracht wird (werden).

Die Expression des ORFs codierend eine Glutamat-Cystein-Ligase wird durch homologe oder heterologe Promotoren erreicht. Ein homologer Promotor bedeutet im Rahmen dieser Erfindung, dass der Promotor gewählt wird, der auch im zum gewählten ORF entsprechenden Wildtyp-Gen die Expression der Glutamat-Cystein-Ligase reguliert. Ein heterologer Promotor bedeutet dagegen, dass ein Promotor gewählt wird, der im entsprechenden Wildtyp-Gen nicht die Expression der Glutamat-Cystein-Ligase reguliert. Im bevorzugten Fall steht zur Expression des GshA-Proteins der ORF unter Kontrolle des gshA-Promotors, d.h. der homologe Promotor wird verwendet. Heterologe Promotoren sind beispielsweise der Promotor des gapA-Gens aus *E. coli,* der Promotor des catB-Gens aus *E. coli,* der Promotor des tufB-Gens aus *E. coli,* der Promotor des mppA-Gens aus *E. coli,* der Promotor des lpp-Gens aus *E. coli* und der Promotor des proC-Gens aus *E. coli.* Weiterhin sind die heterologen lac-, tac-, trc-, lambda-, ara- oder tet-Promotoren dem Fachmann bekannt und können zur heterologen Expression des ORFs codierend eine Glutamat-Cystein-Ligase verwendet werden.

Bevorzugt wird die Expression des ORFs codierend eine Glutamat-Cystein-Ligase in einem Mikroorganismenstamm durch den Promotor des gapA-Gens, durch den Promotor des catB-Gens, durch den Promotor des tufB-Gens, durch den Promotor des mppA-Gens, durch den Promotor des lpp-Gens aus *E. coli* oder durch den Promotor des proC-Gens aus *E. coli* erreicht.

Besonders bevorzugt wird die Expression des ORFs codierend eine Glutamat-Cystein-Ligase auf einem Plasmid in *E. coli* durch den Promotor des tufB-Gens aus *E. coli* erreicht.

Der Mikroorganismenstamm enthält daher bevorzugt mindestens ein Plasmid, das einen der oben genannten Promotoren umfasst, wobei der oder die Promotor(en) sich derart auf dem Plasmid befindet(n), dass sie die Expression des ORFs codierend die Glutamat-Cystein-Ligase steuert(n).

Als Plasmide können bevorzugt alle verfügbaren und gentechnisch zugänglichen DNS-Moleküle verwendet werden, die extrachromosomal im gewählten Mikroorganismenstamm, bevorzugt in *E. coli* repliziert werden und die einen Selektionsmarker umfassen. So können beispielsweise Plasmide mit einer hohen zellulären Kopienzahl in *E. coli* (z.B. Plasmide der pUC-Serie, Plasmide der pQE-Serie, Plasmide der pBluescript-Serie), Plasmide mit einer mittleren Kopienzahl in *E. coli* (z. B. Plasmide der pBR-Serie, Plasmide der pACYC-Serie) oder Plasmide mit einer niedrigen Kopienzahl in *E. coli* (z. B. pSC101 oder pBeloBAC11) eingesetzt werden.

Für *S. cerevisiae* können vom 2 µm-Plasmid abstammende extrachomosomal replizierende Plasmide verwendet werden. Dies können z.B. YEp-Vektoren (yeast episomal plasmids), die ein selektionierbares Markergen enhalten, und eine Kopienzahl von ca. 50 Plasmiden pro Zelle aufweisen. Weiterhin können auch YCp-Vektoren, die zusätzlich eine Centromersequenz für die Segregation der Chromosomen in die Tochterkerne besitzen, verwendet werden. Weiterhin können auch YRp-Vektoren (yeast replicating plasmids) oder auch ARS-Vektoren genannt, die autonom replizierende Sequenzen (ARS-Sequenzen) enthalten, verwendet werden. Diese Plasmide replizieren sich aufgrund des Vorhandenseins von eukaryotischen Replikationsursprüngen autonom, d.h. unabhängig von der chromosomalen DNS.

Weiterhin können in *S. cerevisiae* auch YAC-Vektoren (yeast artificial chromosome), die sich wie eigenständige Chromosomen verhalten, verwendet werden.

Als Plasmide können auch sogenannte Shuttle-Vektoren mit mehreren verschiedenen Replikationsursprüngen, die jeweils in einer anderen Spezies aktiv sind, verwendet werden. Sie sind daher in verschiedenen Mikroorgnaismen vermehrbar. So können z.B. Shuttle-Vektoren, die einen bakteriellen Replikationsursprung aus *E. coli* und einen Replikationsursprung (ARS-Element) aus *S. ce*revisiae besitzen und so in beiden Organismen replizierbar sind, verwendet werden.

Bevorzugt werden Plasmide mit einer mittleren Kopienzahl in *E. coli* (z.B. Plasmide der pBR-Serie, Plasmide der pACYC-Serie) verwendet.

Besonders bevorzugt wird ein Plasmid der pACYC-Serie verwendet.

Das Einbringen eines Plasmids geschieht beispielsweise durch eine gängige Transformationsmethode wie z.B. Elektroporation oder CaCl₂-Methode. Plasmid-tragende Klone werden anschließend über eine Antibiotika-Resistenz selektiert. Dem Fachmann bekannte Selektionsmarker sind beispielsweise das Chloramphenicol-Acetyltransferase-Gen, das Resistenz gegenüber Chloramphenicol vermittelt, das Neomycin-Phosphotransferase-Gen, das Resistenz gegenüber Kanamycin vermittelt, das Tetracyclin-Efflux-Gen, das Resistenz gegenüber Tetracyclin vermittelt oder das β-Lactamase-Gen, das Resistenz gegenüber Ampicillin und Carbenicillin vermittelt.

Alternativ zur Expression von einem Plasmid kann der ORF codierend eine Glutamat-Cystein-Ligase auch in das Chromosom eines Mikroorganismen-Stammes zusätzlich zu einem erfindungsgemäßen Plasmid oder allein integriert werden. Als Integrationsverfahren werden vorzugsweise die dem Fachmann bekannten Systeme mit temperenten Bakteriophagen, integrativen Plasmiden oder die Integration über homologe Rekombination genutzt.

Das Verfahren ist bevorzugt dadurch gekennzeichnet, dass das Fermentationsmedium mindestens 50 - 6000 mg/L L-Tyrosin (L-Tyr) und 50 - 6000 mg/L L-Phenylalanin (L-Phe) enthält.

Das Verfahren ist besonders bevorzugt dadurch gekennzeichnet, dass das Fermentationsmedium mindestens 50 - 3000 mg/L L-Tyrosin (L-Tyr) und 50 - 3000 mg/L L-Phenylalanin (L-Phe) enthält.

Die Kultivierung des erfindungsgemäßen Mikroorganismenstamms erfolgt nach üblichen, dem Fachmann bekannten Verfahren im Schüttelkolben, oder in einem Bioreaktor (Fermenter).

Die Anzucht des erfindungsgemäßen Mikroorganismenstammes im Fermenter erfolgt als kontinuierliche Kultur, als Batch-Kultur oder vorzugsweise als Fed-Batch-Kultur.

Als Kohlenstoff-Quelle dienen vorzugsweise Zucker, Zuckeralkohole oder organische Säuren. Besonders bevorzugt werden im erfindungsgemäßen Verfahren als Kohlenstoff-Quellen Glucose, Lactose, Saccharose oder Glycerin eingesetzt, insbesondere bevorzugt Glucose.

Bevorzugt ist die Dosierung der Kohlenstoff-Quelle in einer Form, die gewährleistet, dass der Gehalt an Kohlenstoff-Quelle im Fermenter während der Fermentation in einem Bereich von 0,1 g/L bis 50 g/L gehalten wird. Besonders bevorzugt ist ein Bereich von 0,1 g/L bis 10 g/L.

Als Stickstoff-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Kontrolle wird während der Fermentation regelmäßig diese Stickstoff-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink, Kupfer und Nickel zugesetzt werden.

Des Weiteren können organische Säuren (z.B. Acetat, Citrat), Aminosäuren (z.B. L-Isoleucin, L-Methionin, L-Phenylalanin, L-Tyrosin, L-Glutamat) und Vitamine (z.B. Vitamin B1, Vitamin B6, Vitamin B12) dem Medium zugesetzt werden.

Als komplexe Nährstoffquellen können z.B. Hefeextrakt, Trypton, Maisquellwasser, Sojamehl oder Malzextrakt zum Einsatz kommen.

Die Inkubationstemperatur zur Anzucht eines erfindungsgemäßen Stammes aus der Familie der Enterobacteriaceae beträgt vorzugsweise 28 - 37 °C, besonders bevorzugt ist eine Inkubationstemperatur von 30 - 32 °C.

Der pH-Wert des Fermentationsmediums liegt während der Fermentation bevorzugt im pH-Bereich von 5,0 bis 8,5, besonders bevorzugt ist ein pH-Wert von 7,0.

Bevorzugt erfolgt die Inkubation des erfindungsgemäßen Mikroorganismenstammes bevorzugt eines Stammes aus der Familie der Enterobacteriaceae besonders bevorzugt eines E. coli-Stammes unter aeroben, anaeroben oder mikroaeroben Kultivierungsbedingungen über einen Zeitraum von 6 h bis 150 h und im Bereich der für den jeweiligen Stamm optimalen Wachstumstemperatur. Besonders bevorzugt sind Kultivierungszeiten zwischen 6 h und 48 h. Die Fermentation wird vorzugsweise unter aeroben oder mikroaeroben Wachstumsbedingungen durchgeführt.

Erfindungsgemäß handelt es sich um ein Verfahren zur fermentativen Produktion von γ-Glu-Tyr und γ-Glu-Phe, wobei der Begriff fermentative Produktion bedeutet, dass mindestens 5 mg γ-Glu-Tyr und 5 mg y-Glu-Phe, bevorzugt mindestens 10 mg γ-Glu-Tyr und 10 mg γ-Glu-Phe und besonders bevorzugt mindestens 20 mg γ-Glu-Tyr und 20 mg γ-Glu-Phe pro Liter Kulturmedium nach 48 Stunden, bevorzugt mindestens 40 Stunden und besonders bevorzugt mindestens 24 Stunden Kultivierungszeit produziert werden.

Bevorzugt werden γ-Glu-Tyr und γ-Glu-Phe nach dem Abtrennen des Fermentationsmediums aus dem Fermentationsmedium aufgereinigt.

Die Abtrennung von γ-Glu-Tyr und γ-Glu-Phe aus der Kultur kann nach dem Fachmann bekannten Verfahren, wie Zentrifugation des Mediums zur Abtrennung der Zellen mit anschließender Gewinnung des γ-Glu-Tyr und γ-Glu-Phe aus dem Fermentationsüberstand erfolgen.

Die Produkte γ-Glu-Tyr und γ-Glu-Phe können nach anschließender Reinigung, Konzentrierung und Trocknung sowie ggf. Formulierung oder Komplexierung erhalten werden.

Der Nachweis und die Quantifizierung des im erfindungsgemäßen Verfahren produzierten γ-Glu-Tyr und γ-Glu-Phe erfolgt beispielsweise mittels einer HPLC-MS-Methode.

Die prinzipielle Vorgehensweise bei der Herstellung von Kokumi-Produkten ergibt sich vorzugsweise wie folgt:
1. Fermenterkultur,
   gefolgt von Abtrennung der Zellen beispielsweise durch Zentrifugation
2. zellfreier Kulturüberstand,
   gefolgt von Steril- und/oder Nanofiltration
3. steriler zellfreier Kulturüberstand
   gefolgt von Trocknung beispielsweise durch Sprüh- und/oder Gefriertrocknung
4. Kokumi-Rohprodukt
   gefolgt von Homogenisation beispielsweise durch Mahlung
5. Kokumi-Produkt

### Beispiele

Die Erfindung wird im Folgenden anhand von Ausführungsbeispielen näher beschrieben, ohne dadurch beschränkt zu werden.

In den Beispielen wurde der in US 2014/0342399 A offenbarte E. coli-Stamm W3110ΔgshB/ptufBₚ-gshA^{ATC}-cysE14-serA2040-orf306 verwendet. Der Stammname bezeichnet den E. *coli* W3110-Stamm (ATCC 27325), dem das gshB-Gen deletiert wurde, wobei die Bakterienzellen das in US 2014/0342399 in Fig. 10 gezeigte Plasmid tragen mit:
- dem gshA-Gen mit dem Startcodon ATG, wobei das Gen unter Kontrolle des tufB-Promotors steht,
- dem serA-Gen (codierend die D-3-Phosphoglycerat-Dehydrogenase) ,
- dem cysE-Gen (codierend die Serin-Acetyltransferase) und
- ORF306 (codierend den Cystein/O-Acetylserin Exporter EamA).

### Beispiel 1: Schüttelkolben-Vorkultur eines γ-Glu-Tyr und γ-Glu-Phe-Produktionsstammes für die Fermentation

100 mL steriles LB-Medium (10 g/L Trypton, 5 g/L Hefeextrakt, 5 g/L NaCl) wurden mit 5 g/L D(+)-Glucose Monohydrat angesetzt und anschließend in einem sterilen 500 mL Erlenmeyerkolben mit Tetracyclin x HCl versetzt (Endkonzentration von Tetracyclin x HCl im Schüttelkolben: 0,01 g/L, sterilfiltriert).

Mit einer Impföse wurden so viele Zellen des Produktionsstammes W3110ΔgshB/ptufBₚ-gshA^{ATG}-cysE14-serA2040-orf306 von der Agarplatte abgenommen und in das supplementierte LB-Medium überführt bis maximal eine schwache Trübung des supplementierten LB-Mediums erkennbar wurde. Diese Vorkultur wurde für 5 h bei 32°C und 130 rpm auf einem Inkubations-Schüttler CERTOMAT^{®} IS (Sartorius Stedium Biotech, Göttingen, Deutschland) kultiviert.

### Beispiel 2: Vorfermenter für die Herstellung von γ-Glu-Tyr und γ-Glu-Phe

Die Vorfermentation erfolgte in einem 2 L DASGIP-Fermenter der Firma Eppendorf (Hamburg, Deutschland).

100 mL LB-Vorkultur aus Beispiel 1 dienten zum Animpfen des 900 mL Produktionsmediums, bestehend aus 30 g/L D(+)-Glucose Monohydrat, 0,03 g/L Pyridoxin x HCl, 0,005 g/L Thiamin x HCl, 0,01 g/L Tetracyclin x HCl, 5 g/L (NH₄)₂SO₄, 0,5 g/L NaCl, 0,9 g/L L-Isoleucin, 0,6 g/L D/L-Methionin, 5 g/L Corn steep solids (z.B. von Sigma-Aldrich), 0,225 g/L CaCl₂ x 2 H₂O, 0,6 g/L MgSO₄ x 7 H₂O, 0,15 g/L Na₂MO₄ x 2 H₂O, 0,3 g/L H₃BO₃, 0,2 g/L CoCl₂ x 6 H₂O, 0,25 g/L CuSO₄ x 5 H₂O, 1,6 g/L MnCl₂ x 4 H₂O, 1,35 g/L ZnSO₄ x 7 H₂O, 0,075 g/L FeSO₄ x 7 H₂O, 1 g/L Na₃-Citrat x 2 H₂O und 1,71 g/L KH₂PO₄. Die Medienbestandteile wurden steril in einem 2 L-Fermenter vorgelegt und mit sterilen Korrekturmitteln (25 % Ammoniak & 6,8 N H₃PO₄) auf pH = 7,0 eingestellt. Während der Kultivierung für 48 h wurden die Temperatur auf 32 °C sowie der pH-Wert bei 7,0 durch das Korrekturmittel (25 % Ammoniak) konstant gehalten. Als Antischaummittel wurde Struktol J673 (Schill + Seilacher, Hamburg) in einer Verdünnung von 1:10 in sterilem Wasser verwendet. Die Kultur wurde mit keimfreier Druckluft mit 100 L/h begast und mit einer Rührerdrehzahl von 450 rpm gerührt. Nach Absinken der Sauerstoffsättigung auf einen Wert von 50 % wurde die Drehzahl über die Steuerungseinheit des Fermenters bis zu einem Wert von 1450 rpm erhöht um 30 % Sauerstoffsättigung zu erhalten. Die Sauerstoffsättigung wurde mit einer pO₂-Sonde bestimmt, die bei 450 rpm auf 100 % Sättigung kalibriert wurde. Sobald der Glucose-Gehalt im Fermenter auf ca. 1-2 g/L abgesunken war, erfolgte die Zudosierung einer 56 % (w/v) D(+)-Glucose Monohydrat-Lösung. Die Glucose-Fütterung erfolgte mit einer Flussrate von 10-15 mL/h wobei die Glucose-Konzentration im Fermenter zwischen 0,1 g/L und 10 g/L konstant gehalten wurde. Die Glucose-Bestimmung wurde mit dem Analysator YSI 7100 MBS (YSI, Yellow Springs, Ohio, USA) durchgeführt.

### Beispiel 3: Fermentative Herstellung von γ-Glu-Tyr und γ-Glu-Phe

Die Herstellung von γ-Glu-Tyr und γ-Glu-Phe in einem 2 L DASGIP-Fermenter der Firma Eppendorf (Hamburg, Deutschland) wurde in zwei unabhängigen Ansätzen durchgeführt.

Die folgende Beschreibung der Versuchsdurchführung gilt für jeden der beiden Ansätze 1 und 2:
100 mL der Vorkultur aus Beispiel 2 dienten zum Animpfen des 900 mL Produktionsmediums, bestehend aus 5 g/L D(+)-Glucose Monohydrat, 0,03 g/L Pyridoxin x HCl, 0,005 g/L Thiamin x HCl, 0,01 g/L Tetracyclin x HCl, 5 g/L (NH₄)₂SO₄, 0,5 g/L NaCl, 0,9 g/L L-Isoleucin, 0,6 g/L D/L-Methionin, 30 g/L Corn steep solids, 0,225 g/L CaCl₂ x 2 H₂O, 0,6 g/L MgSO₄ x 7 H₂O, 0,15 g/L Na₂MO₄ x 2 H₂O, 0,3 g/L H₃BO₃, 0,2 g/L CoCl₂ x 6 H₂O, 0,25 g/L CuSO₄ x 5 H₂O, 1,6 g/L MnCl₂ x 4 H₂O, 1,35 g/L ZnSO₄ x 7 H₂O, 0,075 g/L FeSO₄ x 7 H₂O, 1 g/L Na₃-Citrat x 2 H₂O und 1,71 g/L KH₂PO₄. Die Medienbestandteile wurden steril in einem 2 L-Fermenter vorgelegt und mit sterilen Korrekturmitteln (25 % Ammoniak & 6,8 N H₃PO₄) auf pH = 6,9 eingestellt. Während der Kultivierung für 48 h wurden die Temperatur auf 32 °C sowie der pH-Wert bei 7,0 durch das Korrekturmittel (25 % Ammoniak) konstant gehalten. Als Antischaummittel wurde Struktol J673 (Schill + Seilacher, Hamburg) in einer Verdünnung von 1:10 in sterilem Wasser verwendet. Die Kultur wurde mit keimfreier Druckluft mit 100 L/h begast und mit einer Rührerdrehzahl von 450 rpm gerührt. Nach Absinken der Sauerstoffsättigung auf einen Wert von 50 % wurde die Drehzahl über die Steuerungseinheit des Fermenters bis zu einem Wert von 1450 rpm erhöht um 30 % Sauerstoff Sättigung zu erhalten. Die Sauerstoffsättigung wurde mit einer pO₂-Sonde bestimmt, die bei 450 rpm auf 100 % Sättigung kalibriert wurde. Sobald der Glucose-Gehalt im Fermenter auf ca. 1-2 g/L abgesunken war, erfolgte die Zudosierung einer 56 % (w/v) Glucoselösung. Die Glucose-Fütterung erfolgte mit einer Flussrate von 10-15 mL/h wobei die Glucose-Konzentration im Fermenter zwischen 0,1 g/L und 10 g/L konstant gehalten wurde. Die Glucose-Bestimmung wurde mit dem Analysator YSI 7100 MBS (YSI, Yellow Springs, Ohio, USA) durchgeführt.

Für eine effiziente Herstellung der Produkte γ-Glu-Tyr und γ-Glu-Phe wurde über den Glucose-Feed L-Tyr und L-Phe zudosiert. Die Zufütterung erfolgte 2 h nach Start der Kultivierung mit einer Rate von 10-15 mL/h, wobei die Feedlösung eine Konzentration von 6 g/L L-Tyr und 6 g/L L-Phe besaß.

Zusätzlich zur Glucose-Fütterung wurde Kaliumglutamat und Ammoniumthiosulfat [(NH4)₂S₂O₃] über einen Kombi-Feed zudosiert. Die Zufütterung erfolgte 2 h nach Start der Kultivierung mit einer Rate von 4-4,5 mL/h, wobei die Konzentration von Ammoniumthiosulfat 73 g/L und die Konzentration von Kaliumglutamat 21 g/L in der Feedlösung betrug.

Die Ergebnisse der γ-Glu-Tyr-und γ-Glu-Phe-Fermentationenmit dem Produktionsstamm W3110ΔgshB/ptufBₚ-gshA^{ATG}-cysE14-serA2040-orf306 sind in den Tabellen 1 (1. Ansatz) und 2 (2. Ansatz) zusammengefasst.

**Tabelle 1: Gehalt an γ-Glu-Tyr und γ-Glu-Phe im Kulturüberstand des 1. Ansatzes nach Fermentation des Stammes W3110AgshB/ptufBₚ-gshA^{ATG -}cysE14-serA2040-orf306**

| Produkt | Kultivierungsdauer | |
|---|---|---|
| | 24 h | 48 h |
| γ-Glu-Tyr [mg/L] | 29 | 32 |
| γ-Glu-Phe [mg/L] | 190 | 610 |

**Tabelle 2: Gehalt an γ-Glu-Tyr und γ-Glu-Phe im Kulturüberstand des 2. Ansatzes nach Fermentation des Stammes W3110ΔgshB/ptufBₚ-gshA^{ATG}-cysE14-serA2040-orf306**

| Produkt | Kultivierungsdauer | |
|---|---|---|
| | 24 h | 48 h |
| γ-Glu-Tyr [mg/L] | 23 | 240 |
| γ-Glu-Phe [mg/L] | 22 | 118 |

### Nachweis von γ-Glu-Tyr und γ-Glu-Phe

Die Bestimmung von γ-Glu-Tyr und γ-Glu-Phe erfolgte mittels einer HPLC-MS-Methode. Die Analyse erfolgte auf einer HPLC-Anlage Ultimate 3000 der Firma Thermo Fisher Scientific (Dreieich, Deutschland) gekoppelt mit einem Amazon SL Massenspektrometer der Firma Bruker Daltonik (Bremen, Deutschland). Als Trennsäule diente eine Nucleoshell^{®} Bluebird RP 18-Säule, 2,7 µm (100 mm x 3,0 mm) der Firma Macherey Nagel (Düren, Deutschland). Die Proben wurden in einer Gradientenfahrweise (mobile Phase A: Wasser mit 0,1 % (v/v) Essigsäure, mobile Phase B: Methanol) bei 30 °C und einem Fluss von 0,7 mL/min eluiert. Die massenspektrometrische Detektion erfolgte im ESI-positiv anhand der [M+H]⁺ Ionen (m/z für γ-Glu-Tyr: 311 , m/z für γ-Glu-Phe: 295).

### Beispiel 5: Herstellung eines Kokumi-Produkts

Zur Herstellung eines Kokumi-Produkts wurden 1,5 L einer Fermentation, wie sie in Beispiel 3 beschrieben ist, aufgearbeitet. Nach Zentrifugation zur Abtrennung der Zellen wurde der Überstand sterilfiltriert (Thermo Scientific^{™} Nalgene^{™} Rapid-Flow^{™} 90 mm 0,2 µ Filter Unit, Dreieich, Deutschland) und anschließend in einem Trockenschrank (Binder ED115 E2, Tuttlingen, Deutschland) getrocknet. Zur Homogenisierung wurde das getrocknete Rohprodukt in einer Mühle (IKA^{®} All basic, Staufen, Deutschland) homogenisiert.

## Patentansprüche

1. Verfahren zur fermentativen Produktion von Gamma-Glutamyltyrosin und Gamma-Glutamylphenylalanin, **dadurch gekennzeichnet, dass** ein Bakterienstamm der Familie Enterobacteriaceae in einem Fermentationsmedium kultiviert, das Fermentationsmedium nach der Fermentation von den Zellen abgetrennt und Gamma-Glutamyltyrosin und Gamma-Glutamylphenylalanin aus dem Fermentationsmedium isoliert werden, wobei der Bakterienstamm
a) entweder den im Wildtyp-Genom codierten offenen Leserahmen codierend die Glutamat-Cystein-Ligase und zusätzlich mindestens einen weiteren offenen Leserahmen (ORF) codierend eine Glutamat-Cystein-Ligase enthält
b) oder keinen im Wildtyp-Genom codierten ORF codierend eine Glutamat-Cystein-Ligase, aber mindestens einen weiteren ORF codierend eine Glutamat-Cystein-Ligase enthält und
wobei der Bakterienstamm defizient in der Glutathion-Synthethase ist, d.h. vom entsprechenden Gen wird keine funktionelle Glutathion-Synthethase produziert und
wobei das Fermentationsmedium mindestens 50 mg/L L-Tyrosin und 50 mg/L L-Phenylalanin enthält.

2. Verfahren gemäß Anspruch 1, **dadurch gekennzeichnet, dass** es sich beim Bakterienstamm um einen Stamm der Art *Escherichia coli* handelt.

3. Verfahren gemäß einem oder mehreren der Ansprüche 1 oder 2, **dadurch gekennzeichnet, dass** es sich beim weiteren offenen Leserahmen codierend eine Glutamat-Cystein-Ligase um SEQ ID NO. 1 oder Homologe dieser Sequenz handelt,
wobei Homologe solche ORFs sind, die eine Sequenzidentität größer 70 % zu SEQ ID NO. 1 besitzen und das von dieser DNS-Sequenz exprimierte Protein Glutamat-Cystein-Ligase-Funktion aufweist.

4. Verfahren gemäß einem oder mehreren der Ansprüche 1 bis 3, **dadurch gekennzeichnet, dass** der Bakterienstamm mindestens ein Plasmid enthält, das mindestens einen ORF codierend eine Glutamat-Cystein-Ligase unter Kontrolle eines funktionellen Promotors enthält.

5. Verfahren gemäß einem oder mehreren der Ansprüche 1 bis 4, **dadurch gekennzeichnet, dass** nach dem Abtrennen des Fermentationsmediums Gamma-Glutamyltyrosin und Gamma-Glutamylphenylalanin aus dem Fermentationsmedium aufgereinigt werden.

## Claims

1. Process for fermentative production of gamma-glutamyltyrosine and gamma-glutamylphenylalanine, **characterized in that** a bacterial strain of the Enterobacteriaceae family is cultured in a fermentation medium, the fermentation medium is removed from the cells after the fermentation, and gamma-glutamyltyrosine and gamma-glutamylphenylalanine are isolated from the fermentation medium,
wherein the bacterial strain
a) either contains the open reading frame encoded in the wild-type genome that encodes glutamate-cysteine ligase and, in addition, at least one further open reading frame (ORF) encoding a glutamate-cysteine ligase
b) or contains no ORF encoded in the wild-type genome that encodes a glutamate-cysteine ligase, but at least one further ORF encoding a glutamate-cysteine ligase, and
wherein the bacterial strain is deficient in glutathione synthetase, i.e., no functional glutathione synthetase is produced by the corresponding gene, and
wherein the fermentation medium contains at least 50 mg/L L-tyrosine and 50 mg/L L-phenylalanine.

2. Process according to Claim 1, **characterized in that** the bacterial strain is a strain of the species *Escherichia coli.*

3. Process according to one or more of Claims 1 and 2, **characterized in that** the further open reading frame encoding a glutamate-cysteine ligase is SEQ ID NO.1 or homologs of this sequence,
wherein homologs are those ORFs that have a sequence identity greater than 70% in relation to SEQ ID NO.1 and the protein expressed by this DNA sequence has a glutamate-cysteine ligase function.

4. Process according to one or more of Claims 1 to 3, **characterized in that** the bacterial strain contains at least one plasmid which contains at least one ORF encoding a glutamate-cysteine ligase under the control of a functional promoter.

5. Process according to one or more of Claims 1 to 4, **characterized in that** gamma-glutamyltyrosine and gamma-glutamylphenylalanine are purified from the fermentation medium after the removal of the fermentation medium.

## Revendications

1. Procédé de production par fermentation de gamma-glutamyltyrosine et de gamma-glutamylphénylalanine, **caractérisé en ce qu'**une souche bactérienne de la famille des Enterobacteriaceae est cultivée dans un milieu de fermentation, le milieu de fermentation est séparé des cellules après la fermentation et la gamma-glutamyltyrosine et la gamma-glutamylphénylalanine sont isolées du milieu de fermentation,
la souche bactérienne
a) soit contenant le cadre de lecture ouvert codé dans le génome de type sauvage codant pour la glutamate-cystéine-ligase et de plus au moins un autre cadre de lecture ouvert (ORF) codant pour une glutamate-cystéine-ligase
b) soit ne contenant aucun ORF codé dans le génome de type sauvage codant pour une glutamate-cystéine-ligase, mais contenant au moins un autre ORF codant pour une glutamate-cystéine-ligase et
la souche bactérienne étant déficiente en glutathion-synthétase, c'est-à-dire qu'aucune glutathion-synthétase fonctionnelle n'est produite par le gène correspondant, et le milieu de fermentation contenant au moins 50 mg/L de L-tyrosine et 50 mg/L de L-phénylalanine.

2. Procédé selon la revendication 1, **caractérisé en ce que** la souche bactérienne est une souche de l'espèce *Escherichia coli.*

3. Procédé selon l'une quelconque ou plusieurs des revendications 1 et 2, **caractérisé en ce que**, dans le cas du cadre de lecture ouvert supplémentaire codant pour une glutamate-cystéine-ligase, il s'agit de la SEQ ID NO. 1 ou d'homologues de cette séquence, les homologues étant de tels ORF qui possèdent une identité de séquence supérieure à 70 % avec la SEQ ID NO. 1 et la protéine exprimée par cette séquence d'ADN présentant une fonction de glutamate-cystéine-ligase.

4. Procédé selon l'une quelconque ou plusieurs des revendications 1 à 3, **caractérisé en ce que** la souche bactérienne contient au moins un plasmide qui contient au moins un ORF codant pour une glutamate-cystéine-ligase sous le contrôle d'un promoteur fonctionnel.

5. Procédé selon l'une quelconque ou plusieurs des revendications 1 à 4, **caractérisé en ce qu'**après la séparation du milieu de fermentation, la gamma-glutamyltyrosine et la gamma-glutamylphénylalanine sont purifiées à partir du milieu de fermentation.
